(19) Europäisches Patentamt
European Patent Office
Office européen des brevets

(11) **EP 2 946 020 B1**

(12) **EUROPEAN PATENT SPECIFICATION**

(45) Date of publication and mention
of the grant of the patent:
**22.11.2017 Bulletin 2017/47**

(51) Int Cl.:
***C12Q 1/68*** (2006.01)   ***G01N 21/65*** (2006.01)

(21) Application number: **14700782.7**

(22) Date of filing: **17.01.2014**

(86) International application number:
**PCT/GB2014/050130**

(87) International publication number:
**WO 2014/111723 (24.07.2014 Gazette 2014/30)**

(54) **DROPLET-BASED SEQUENCING METHOD**

TRÖPFCHENBASIERTES SEQUENZIERUNGSVERFAHREN

PROCÉDÉ DE SÉQUENÇAGE À BASE DE GOUTTELETTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.01.2013 GB 201300957**

(43) Date of publication of application:
**25.11.2015 Bulletin 2015/48**

(73) Proprietor: **Base4 Innovation Ltd**
**Cambridge, Cambridgeshire CB3 0FA (GB)**

(72) Inventors:
• **NATALE, Alessandra**
**Cambridge**
**Cambridgeshire CB3 0FA (GB)**
• **SOARES, Bruno Flavio Nogueira de Sousa**
**Cambridge**
**Cambridgeshire CB3 0FA (GB)**
• **FRAYLING, Cameron Alexander**
**Cambridge**
**Cambridgeshire CB3 0FA (GB)**
• **AMASIO, Michele**
**Cambridge**
**Cambridgeshire CB3 0FA (GB)**
• **ISAAC, Thomas Henry**
**Cambridge**
**Cambridgeshire CB3 0FA (GB)**

(74) Representative: **Lau, Sarah Jane et al**
**Kilburn & Strode LLP**
**Lacon London**
**84 Theobalds Road**
**London WC1X 8NL (GB)**

(56) References cited:
**WO-A1-2005/035791     US-A1- 2003 138 831**
**US-A1- 2003 187 237     US-A1- 2009 280 475**
**US-A1- 2012 164 633**

• **STEVEN E. J. BELL ET AL: "Surface-Enhanced Raman Spectroscopy (SERS) for Sub-Micromolar Detection of DNA/RNA Mononucleotides", JOURNAL OF THE AMERICAN CHEMICAL SOCIETY, vol. 128, no. 49, 1 December 2006 (2006-12-01), pages 15580-15581, XP055108992, ISSN: 0002-7863, DOI: 10.1021/ja066263w**

**Description**

The present invention relates to a method for determining the sequence of nucleotide bases in polynucleotides derived from, for example, naturally occurring RNA or DNA.

Next generation sequencing of genetic material is already making a significant impact on the biological sciences in general and medicine in particular as the unit cost of sequencing falls in line with the coming to market of faster and faster sequencing machines. Thus, in one such machine, a double-stranded DNA analyte is first broken down into a plurality of smaller polynucleotide fragments each of which is first adenylated on both ends of one strand so that a single-stranded first oligonucleotide can be bound to both ends of its compliment by hybridisation to the unpaired adenine base. The treated fragments so obtained are then size-selected and captured on a surface coated with bound single-stranded second oligonucleotides which themselves are the sequence compliment of the first so that in effect a library of surface-bound double-stranded fragments can be created by further hybridisation. In a subsequent clustering step, these library components are then clonally amplified millions of times on the surface using extension and isothermal bridging reactions to utilise unused second oligonucleotides. This, in effect, creates a dense concentration of the polynucleotide fragment bound to the surface through one of its strands. The unbound complimentary strand of each fragment is then removed to leave bound single-stranded fragments ready for sequencing. In the sequencing stage, each of these single-stranded fragments is primed and its complimentary strand recreated by extension using the polymerase chain reaction and a mixture of the four characteristic nucleotide bases of DNA in dideoxynucleotidetriphosphate (ddNTP) form. Each ddNTP type is end-blocked with a moiety which is labelled with a different fluorophore fluorescing at a different wavelength. The extension reaction then takes the form of a cycle of three steps; first the relevant ddNTP is bounded to the growing strand; secondly the nucleotide base it contains is identified by illuminating the sample and detecting the wavelength of the fluorescence and finally the end block and its associated fluorophore are removed to allow the next extension event to occur. By this means, the sequence of the complimentary strand can be built up base-by-base. It will be appreciated that, whilst this approach can be highly automated and can generate sequence reads of high accuracy, its speed of operation is limited by the rate of the extension cycle. Thus, in practice, use of the technology, tends to involve parallel processing of relatively short polynucleotide fragments and assembly of the whole sequence from the various reads obtained therefrom. This in itself can lead to computational complexities and the potential introduction of errors.

More recently efforts have been made to develop direct sequencing methods. For example, our co-pending application WO 2009/030953 discloses a new fast sequencer in which *inter alia* the sequence of nucleotide bases or base pairs in a single- or double-stranded polynucleotide sample (e.g. naturally occurring RNA or DNA) is read by translocating the same through a nano-perforated substrate provided with plasmonic nanostructures juxtaposed within or adjacent the outlet of the nanopores. In this device, the plasmonic nanostructures define detection windows (essentially an electromagnetic field) within which each nucleotide base (optionally labelled) is in turn induced to fluoresce or Raman scatter photons in a characteristic way by interaction with incident light. The photons so generated are then detected remotely, multiplexed and converted into a data stream whose information content is characteristic of the nucleotide base sequence associated with the polynucleotide. This sequence can then be recovered from the data stream using computational algorithms embodied in corresponding software programmed into a microprocessor integral therewith or in an ancillary computing device attached thereto. Further background on the use of plasmonic nanostructures and their associated resonance characteristics can be found in for example Adv. Mat. 2004, 16(19) pp. 1685-1706.

Another apparatus for fast sequencing polynucleotides is described, for example, in US 6627067, US 6267872 and US 6746594. In its simplest form, this device employs electrodes, instead of plasmonic nanostructures, to define the detection window across the substrate or in or around the outlet of the nanopore. A potential difference is then applied across the electrodes and changes in the electrical characteristics of the ionic medium flowing therebetween, as a consequence of the electrophoretic translocation of the polynucleotide and associated electrolyte through the nanopore, is measured as a function of time. In this device, as the various individual nucleotide bases pass through the detection window they continuously block and unblock it causing 'events' which give rise to characteristic fluctuations in current flow or resistivity. These fluctuations are then used to generate a suitable data stream for analysis as described above.

The patent application WO2005/035791 A1 concerns nucleic acid sequencing by enhanced Raman spectroscopy.

The generation of stable droplet streams, especially microdroplet streams, is another developing area of technology that already has applications in molecular biology. For example, US7708949 discloses a novel microfluidic method for generating stable water droplets in oil whilst for example US2011/0250597 describes utilisation of this technology to generate microdroplets containing a nucleic acid template (typically a polynucleotide DNA or RNA fragment) and a plurality of primer pairs that enable the template to be amplified using the polymerase chain reaction. Other patent applications relating to the field include JP2004/290977, JP2004/351417, US2012/0122714, US2011/0000560, US2010/01376163, US2010/0022414 and US2008/0003142.

We have now developed a new method of determining the sequence of nucleotide bases in a polynucleotide analyte which is different from those described above. In one embodiment of this method, individual single nucleotide base-containing droplets, which when taken together comprise a droplet stream whose ordering suitably corresponds to that of the analyte, are characterised by Raman spectroscopy. A feature of the method is that each droplet comprises not only the single nucleotide base but also a colloid of metal particles capable of undergoing plasmon resonance when stimulated by electromagnetic radiation of the correct wavelength. This plasmon resonance works to create a strong electromagnetic field around the particles and within the droplet enhancing the Raman scattering of incident light by the particular nucleotide base contained therein.

Thus, according to the present invention, there is provided a method for determining the sequence of nucleotide bases in a polynucleotide analyte characterised by the steps of:

a. generating a stream of droplets at least some of which comprise both (1) a single nucleotide base and (2) colloidal metal particles capable of undergoing plasmon resonance wherein the ordering of the single nucleotide bases in the droplet stream corresponds to the sequence in the analyte, and

b. irradiating each droplet with electromagnetic radiation to cause (1) the colloidal metal particles contained therein to undergo plasmon resonance and (2) the nucleotide base also contained therein to Raman scatter light at one or more wavelengths characteristic of its type.

In step (a) of the method of the present invention a stream of droplets at least some of which comprise both a single nucleotide base and colloid of metal particles capable of undergoing plasmon resonance is generated. Typically the droplets are aqueous and the droplet stream is maintained in an immiscible carrier solvent such as a hydrocarbon or silicone oil. To avoid the risk that a given droplet contains more than one single nucleotide base it is preferred to incorporate the nucleotide bases at a rate such that each filled droplet is separated by from 1 to 20 preferably 2 to 10 empty ones. Thereafter the stream of filled droplets and the solvent is caused to flow along a flow path, suitably a microfluidic flow path, at a rate and in a manner such that the droplets are maintained in a discrete state and do not have the opportunity to coalesce with one another. Suitably the droplets employed have a diameter less than 100 microns, preferably less than 50 microns more preferably less than 20 microns and even more preferably less than 15 microns. Most preferably of all, their diameters are in the range 2 to 20 microns. Optionally, the droplets may contain additional components to prevent the undesirable formation of emulsions or improve surface tension. Typically the single nucleotide bases are those derived from a naturally occurring polynucleotide analyte preferably naturally occurring RNA or DNA although others not usually encountered in nature or synthetic analogues can also be employed.

In one embodiment of step (a) the ordered droplet stream is created by introducing a stream of single nucleotide bases into a corresponding stream of droplets comprised only of the aqueous colloid of the metal particles and any optional additives. This introduction can be done, for example, by creating a second ordered droplet stream containing the single nucleotide bases and causing corresponding droplets in the two streams to coalesce sequentially into a single stream of larger droplets containing both components. Alternatively, the single nucleotide bases can be captured and injected base by base into sequential droplets comprising the colloid.

As regards the stream of single nucleotide bases itself, this may be suitably generated from the polynucleotide analyte by, for example, the action of an exonuclease thereon in a flowing aqueous medium which continuously removes each nucleotide base as it is liberated. Alternatively, the single nucleotide bases can be generated from the polynucleotide analyte by pyrophosphorolysis in the presence of high levels of pyrophosphate anion under conditions where the liberated nucleotide bases are continuously removed from the reaction zone to avoid establishment of the reverse conventional polymerase chain reaction. This pyrophosphorolysis reaction is preferably carried out at a temperature in the range 20 to 90°C, preferably 50 to 75°C, in the presence of an aqueous reaction medium comprising an enzyme. Preferably it is carried out under conditions of non-equilibrium flow so that the single nucleotides bases are continually removed from the reaction zone. Most preferably, the reaction is carried out by causing an aqueous buffered medium containing the enzyme to continuously flow over the surface to which the analyte is bound. The enzyme used in this second embodiment is suitably one which can cause progressive 3'-5' pyrophosphorolytic degradation of the analyte to yield deoxyribonucleotidetriphosphates with high selectivity and at a reasonable reaction rate. Suitably this degradation reaction is carried out as quickly as possible for example at rates in the range 1 to 50 or more nucleotides per second. Further information about the pyrophosphorolysis reaction as applied to polynucleotides can be found for example in J. Biol. Chem. 244 (1969) pp. 3019-3028 the relevant subject-matter of which is incorporated herein by reference. The enzyme is suitably selected from the group consisting of those polymerases which show essentially neither exo- nor endonuclease activity under the reaction conditions. Examples of polymerases which can be advantageously used include, but are not limited to, the prokaryotic pol 1 enzymes or enzyme derivatives obtained from bacteria such as *Escherichia coli* (e.g. Klenow fragment polymerase), *Thermus aquaticus* (e.g. *Taq* Pol) and *Ba-*

*cillus stearothermophilus, Bacillus caldovelox* and *Bacillus caldotenax.* Suitably, the pyrophosphorolytic degradation is carried out in the presence of a medium which further comprises pyrophosphate anion and magnesium cations in preferably millimolar concentrations.

Preferably, the metal particles comprising the colloid are made of copper, silver, gold or suitable composites of these metals with each other or other metals. These particles may be of any shape and suitably have an average maximum dimension of less than 2500nm preferably less than 150 nm, for example in the range 5 to 100nm most preferably 10 to 50nm. Suitably the concentration of metal particles in the aqueous droplets is as high as possible consistent (1) with the colloid remaining stable under its conditions of use and (2) the plasmon resonance characteristics of the metal particles being not adversely affected. In a preferred embodiment, a colloid of substantially spherical gold particles is employed which can conveniently be made by the reduction of gold salt such as chloroauric acid with for example a citrate salt, hydroquinone or a sugar such as glucose. The methods for carrying out this reaction are well-known in the art; indeed gold colloids produced in this way are readily available commercially. Typically, the surfaces of the metal particles are surrounded by an ionic sheath which repels like metal particles and prevents agglomeration. For example, in the case where the particles are made by sodium citrate reduction of an auric salt, the associated surface charge will be negative (from absorbed citrate anion) with charge-balancing sodium cations present in the surrounding aqueous phase. Whilst this surface charge can be used to help bind the nucleotide base to the particle it is envisaged that the surface can also be further chemically modified to provide specific and stronger binding sites for the nucleotide base if so desired.

In step (b) of the process each droplet produced in step (a) is irradiated with electromagnetic radiation to cause the metal particles to undergo plasmon resonance. Suitably the source of electromagnetic radiation will be a high intensity monochromatic light source such as that produced by a laser. The purpose of this is to cause enhancement of the Raman scattering signal. In one embodiment this irradiation may be carried out dynamically by passing a microfluidic stream of droplets in oil through a 'detection window' where each droplet is exposed to the radiation in turn for a relatively short period of time. Alternatively and preferably the droplets from step (a) are suitably tracked to a capture location where they can be held more or less indefinitely, for example by physical confinement in a chamber or channel. The advantage of this method is that the droplets can be potentially irradiated for longer periods and for the user to be satisfied that a given droplet actually contains a nucleotide base. This method is especially useful where the droplet flow is high for example in the range 50 to 3000 droplets per second preferably 100 to 2000.

The Raman scattered light produced by the nucleotide bases is detected at one or more wavelengths characteristic of the various nucleotide base types from which the analyte is constituted. Thus if the analyte is DNA, one possibility is that four wavelengths are chosen each of which is uniquely characteristic of a vibrational mode of the constituent adenine, thymine, guanine and cytosine bases. The detection of additional wavelengths can also be used to allow the characterisation of modified nucleotide bases, for example methylation. In another embodiment, each nucleotide may also be detected at multiple wavelengths to ensure they are detected reliably. Typically the wavelengths at which this detection occurs have the value $\lambda_d$ wherein:

$$\lambda_d = \lambda_e + \lambda_b \text{ (Stokes shift)}$$

$$\lambda_d = \lambda_e - \lambda_b \text{ (Anti-Stokes shift)}$$

$\lambda_e$ is the wavelength of the incident light (typically occurring in the visible or near ultra-violet) and $\lambda_b$ is the wavelength of the characteristic vibrational mode of the base (typically occurring in the infra-red). In one embodiment detection is carried out on Stokes shifted scattered light. Typically a photodetector or photon counter is used to detect and quantify the amount of Raman scattered light. In another embodiment of the present invention the carrying out of step (b) occurs when the nucleotide base has become attached to at least one of the metal particles so that the Raman scattered light is amplified by the so-called Surface Enhanced Raman Spectroscopic effect (SERS). Alternatively, it is possible to detect Anti-Stokes shifted scattered light but in such an approach the single nucleotide base needs to be first pumped up to a low-lying excited vibrational state by a second source of electromagnetic radiation of the correct wavelength. In all the embodiments described above, where the droplets are tracked to a given location, the various locations can be studied sequentially to yield an output which can be computationally converted into a sequence listing.

Typically the vibrational modes of the nucleotide bases which are detected by Raman scattering are those associated with the in-phase ring breathing vibration. This occurs at frequency shifts of 485 to 505 $\text{cm}^{-1}$ for thymine, 675 to 690 $\text{cm}^{-1}$ for guanine, 775 to 800 $\text{cm}^{-1}$ for cytosine, and 724 to 732 $\text{cm}^{-1}$ for adenine.

The droplet sequencing method of the present invention is now illustrated with reference to a device in which the Figure schematically illustrates a sequencing device in which aqueous droplets each containing a single nucleotide base and colloidal gold are created and detected.

An aqueous stream 1 containing single nucleotide bases obtained from a 100 nucleotide base polynucleotide analyte derived from human DNA is caused to flow through a ten micron diameter microfluidic tube.

The single nucleotide bases themselves are in the form of deoxyribonucleotidemonophosphates prepared by digesting the analyte, attached to the internal surface of the tube, with Klenow fragment exonuclease. The order of single nucleotide bases in the stream thus corresponds to their sequence in the analyte. 1 emerges from a droplet head 2 into a first chamber 3 where it is contacted with one or more streams of light silicone oil 4. The velocities of these streams are chosen to avoid turbulent mixing and to create substantially aqueous spherical droplets 5 suspended in the oil each having a diameter of approximately eight microns. A stream of 5 is then carried forward along a second microfluidic tube of the same diameter at a rate of 1000 droplets per second to a second chamber 6 into which a second stream of five micron aqueous spherical droplets 7 is also fed using a second droplet head 8. Droplets 7 comprise an aqueous colloid of spherical gold particles (average maximum dimension 10nm, concentration 5mg/ml) and are caused to coalesce in a sequential fashion with 5 to form enlarged aqueous droplets 9 approximately nine microns in diameter. Droplets 9 are then delivered to a container 10 in which their progress is tracked until they reach one of an array of sites 11a where they are held (11b) until they are analysed. Typically, analysis occurs after all the sites are filled or all of the analyte is consumed.

Each droplet in the array is then illuminated in the correct order with high intensity laser light (for example the 532nm wavelength light from a Nd:YAG laser) and the Raman scattered Stoke-shifted light is detected by a photodetector operating at the wavelengths shifts from the incident laser light mentioned above characteristic of the four nucleotide base types of DNA. From the information received the single nucleotide base is identified in each droplet and nil responses from empty droplets rejected. The results are then processed by a computer programmed to recreate the original nucleotide base sequence of the analyte. If so desired multiple cycles of illumination and detection can be performed across the array of droplets at various intervals which can be averaged to improve the single to noise ratio and therefore the reliability of the results.

## Claims

1. A method for determining the sequence of nucleotide bases in a polynucleotide analyte **characterised by** the steps of:

   a. generating a stream of droplets at least some of which comprise both (1) a single nucleotide base and (2) colloidal metal particles capable of undergoing plasmon resonance wherein the single nucleotide bases are prepared from the polynucleotide analyte and the ordering of the single nucleotide bases in the droplet stream corresponds to the sequence in the analyte; and

   b. irradiating each droplet with electromagnetic radiation to cause (1) the colloidal metal particles contained therein to undergo plasmon resonance and (2) the nucleotide base also contained therein to Raman scatter light at one or more wavelengths characteristic of its type.

2. A method as claimed in claim 1 **characterised in that** the stream of droplets used in step (a) is generated by inserting the single nucleotide bases into a stream of droplets comprising an aqueous colloid of metal particles.

3. A method as claimed in claim 2 **characterised in that** the stream of droplets used in step (a) is generated by coalescing a corresponding stream of aqueous droplets containing the single nucleotide base with the stream of droplets comprising an aqueous colloid of the metal particles.

4. A method as claimed in any of the preceding claims **characterised in that** single nucleotide bases are prepared from the polynucleotide analyte by either the action of an exonuclease, the exonuclease activity of a polymerase, or pyrophosphorolysis.

5. A method as claimed in any of the preceding claims **characterised in that** the metal is selected from copper, silver or gold.

6. A method as claimed in any one of the preceding claims **characterised in that** the metal particles have an average maximum dimension in the range 5 to 100nm.

7. A method as claimed in any one of the preceding claims **characterised in that** the metal particles have an average maximum dimension in the range 10 to 50nm.

8. A method as claimed in any one of the preceding claims **characterised in that** the surface of the metal particles is chemically modified to bind to the nucleotide base.

9. A method as claimed in any one of the preceding claims **characterised in that** polynucleotide analyte is RNA or DNA and each single nucleotide base is selected from the group consisting of thymine, guanine, cytosine, adenine and uracil.

10. A method as claimed in any one of the preceding claims **characterised in that** the electromagnetic radiation used in step (b) is laser light.

11. A method as claimed in any one of the preceding claims **characterised in that** the Raman scattered light is Stokes shifted light.

**12.** A method as claimed in any of the preceding claims wherein the Raman scattered light is anti-Stokes shifted light generated using excitation of the nucleotide base with a second source of electromagnetic radiation.

**13.** A method as claimed in any one of the preceding claims **characterised in that** the single nucleotide is chemically or physically bound to the surface of at least one metal particle.

**14.** A method as claimed in any one of the preceding claims **characterised in that** the droplets are tracked to a location where they are confined and where detection of the Raman scattered light occurs at that location.

**Patentansprüche**

**1.** Verfahren zur Bestimmung der Sequenz von Nukleotidbasen in einem Polynukleotid-Analyten, **gekennzeichnet durch** die folgenden Schritte:

a. Erzeugen eines Stroms von Tröpfchen, von denen zumindest einige (1) eine einzelne Nukleotidbase und (2) kolloidale Metallpartikel umfassen, die einer Plasmonresonanz unterliegen können, wobei die einzelnen Nukleotidbasen aus dem Polynukleotid-Analyten hergestellt worden sind und die Reihenfolge der einzelnen Nukleotidbasen in dem Tröpfchenstrom der Sequenz in dem Analyten entspricht, und

b. Bestrahlen jedes Tröpfchens mit elektromagnetischer Strahlung, um zu bewirken, dass (1) die darin enthaltenen kolloidalen Metallpartikel einer Plasmonresonanz unterliegen und (2) die ebenfalls darin enthaltene Nukleotidbase Licht bei einer oder mehreren für ihren Typ charakteristischen Wellenlängen Ramanstreut.

**2.** Verfahren nach Anspruch 1, **dadurch gekennzeichnet, dass** der in Schritt (a) verwendete Strom von Tröpfchen durch Einbringen der einzelnen Nukleotidbasen in einen Strom von Tröpfchen, die ein wässriges Kolloid von Metallpartikeln umfassen, erzeugt wird.

**3.** Verfahren nach Anspruch 2, **dadurch gekennzeichnet, dass** der in Schritt (a) verwendete Strom von Tröpfchen durch Koaleszieren eines entsprechenden Stroms von wässrigen Tröpfchen, die die einzelne Nukleotidbase enthalten, mit dem Strom von Tröpfchen, die ein wässriges Kolloid der Metallpartikel umfassen, erzeugt wird.

**4.** Verfahren nach einem der vorhergehenden Ansprüche, **dadurch gekennzeichnet, dass** einzelne Nukleotidbasen aus dem Polynukleotid-Analyten entweder durch Einwirkung einer Exonuklease, die Exonuklease-Aktivität einer Polymerase oder Pyrophosphorolyse hergestellt werden.

**5.** Verfahren nach einem der vorhergehenden Ansprüche, **dadurch gekennzeichnet, dass** das Metall aus Kupfer, Silber oder Gold ausgewählt ist.

**6.** Verfahren nach einem der vorhergehenden Ansprüche, **dadurch gekennzeichnet, dass** die Metallpartikel eine durchschnittliche maximale Abmessung im Bereich von 5 bis 100 nm besitzen.

**7.** Verfahren nach einem der vorhergehenden Ansprüche, **dadurch gekennzeichnet, dass** die Metallpartikel eine durchschnittliche maximale Abmessung im Bereich von 10 bis 50 nm aufweisen.

**8.** Verfahren nach einem der vorhergehenden Ansprüche, **dadurch gekennzeichnet, dass** die Oberfläche der Metallpartikel chemisch so modifiziert ist, dass sie an die Nukleotidbase binden.

**9.** Verfahren nach einem der vorhergehenden Ansprüche, **dadurch gekennzeichnet, dass** der Polynukleotid-Analyt RNA oder DNA ist und jede einzelne Nukleotidbase aus der aus Thymin, Guanin, Cytosin, Adenin und Uracil bestehenden Gruppe ausgewählt ist.

**10.** Verfahren nach einem der vorhergehenden Ansprüche, **dadurch gekennzeichnet, dass** die in Schritt (b) verwendete elektromagnetische Strahlung Laserlicht ist.

**11.** Verfahren nach einem der vorhergehenden Ansprüche, **dadurch gekennzeichnet, dass** das Raman-Streuungslicht Stokes-Verschiebungs-Licht ist.

**12.** Verfahren nach einem der vorhergehenden Ansprüche, wobei es sich bei dem Raman-Streuungslicht um Anti-Stokes-Verschiebungs-Licht handelt, das unter Verwendung von Anregung der Nukleotidbase mit einer zweiten Quelle von elektromagnetischer Strahlung erzeugt wird.

**13.** Verfahren nach einem der vorhergehenden Ansprüche, **dadurch gekennzeichnet, dass** das einzelne Nukleotid chemisch oder physikalisch an die Oberfläche mindestens eines Metallpartikels gebunden ist.

**14.** Verfahren nach einem der vorhergehenden Ansprüche, **dadurch gekennzeichnet, dass** die Tröpfchen zu einem Ort verfolgt werden, an den sie eingeschlossen werden und wobei der Nachweis des Raman-Streuungslichts an diesem Ort erfolgt.

**Revendications**

1. Procédé destiné à déterminer la séquence des bases nucléotidiques dans un analyte de polynucléotides, **caractérisé par** les étapes consistant à :

   a. générer un flux de gouttelettes dont au moins quelques-unes d'entre elles comprennent à la fois (1) une base nucléotidique simple et (2) des particules colloïdales de métal étant capables de subir une résonance plasmonique, dans lequel les bases nucléotidiques simples sont préparées à partir de l'analyte de polynucléotides et le rangement des bases nucléotidiques simples dans le flux de gouttelettes correspond à la séquence dans l'analyte ; et
   b. irradier chaque gouttelette avec un rayonnement électromagnétique pour faire en sorte que (1) les particules colloïdales de métal contenues dans celle-ci subissent une résonance plasmonique, et (2) que la base nucléotidique également contenue dans celle-ci diffuse la lumière, par diffusion Raman, à une ou plusieurs longueur(s) d'onde caractéristique(s) de son type.

2. Procédé, selon la revendication 1, **caractérisé en ce que** le flux de gouttelettes utilisé dans l'étape (a) est généré en insérant les bases nucléotidiques simples dans un flux de gouttelettes qui comprend un colloïde aqueux de particules métalliques.

3. Procédé, selon la revendication 2, **caractérisé en ce que** le flux de gouttelettes utilisé dans l'étape (a) est généré en rendant coalescents un flux correspondant de gouttelettes aqueuses, contenant la base nucléotidique simple, avec le flux de gouttelettes comprenant un colloïde aqueux de particules métalliques.

4. Procédé, selon l'une quelconque des revendications précédentes, **caractérisé en ce que** des bases nucléotidiques simples sont préparées à partir de l'analyte de polynucléotides par : l'action d'une exonucléase, l'activité de nucléase d'une polymérase ou une pyrophosphorolyse.

5. Procédé, selon l'une quelconque des revendications précédentes, **caractérisé en ce que** le métal est choisi parmi le cuivre, l'argent ou l'or.

6. Procédé, selon l'une quelconque des revendications précédentes, **caractérisé en ce que** les particules de métal ont une dimension moyenne maximum comprise dans la gamme des 5 à 100nm.

7. Procédé, selon l'une quelconque des revendications précédentes, **caractérisé en ce que** les particules de métal ont une dimension moyenne maximum comprise dans la gamme des 10 à 50nm.

8. Procédé, selon l'une quelconque des revendications précédentes, **caractérisé en ce que** la surface des particules de métal est chimiquement modifiée pour se lier à la base nucléotidique.

9. Procédé, selon l'une quelconque des revendications précédentes, **caractérisé en ce que** l'analyte de polynucléotides est de l'ARN ou de l'ADN et que chaque base nucléotidique simple est choisie dans le groupe constitué par la thymine, la guanine, la cytosine, l'adénine et l'uracile.

10. Procédé, selon l'une quelconque des revendications précédentes, **caractérisé en ce que** le rayonnement électromagnétique utilisé dans l'étape (b) est la lumière laser.

11. Procédé, selon l'une quelconque des revendications précédentes, **caractérisé en ce que** la lumière diffusée de Raman est une lumière à décalage de Stokes.

12. Procédé, selon l'une quelconque des revendications précédentes, **caractérisé en ce que** la lumière diffusée de Raman est une lumière à décalage anti-Stokes générée en utilisant une excitation de la base nucléotidique avec une deuxième source de rayonnement électromagnétique.

13. Procédé, selon l'une quelconque des revendications précédentes, **caractérisé en ce que** le nucléotide simple est lié chimiquement ou physiquement à la surface d'au moins une particule de métal.

14. Procédé, selon l'une quelconque des revendications précédentes, **caractérisé en ce que** les gouttelettes sont suivies jusqu'à un emplacement où elles sont confinées et où une détection de la lumière diffusée de Raman a lieu à cet emplacement-là.

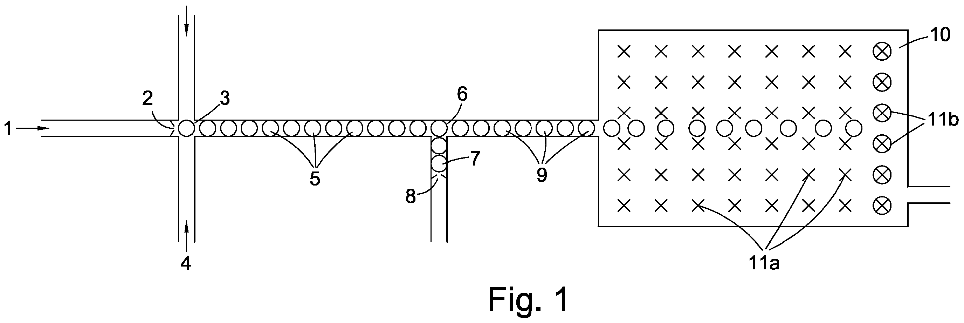

Fig. 1

EP 2 946 020 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009030953 A
- US 6627067 B
- US 6267872 B
- US 6746594 B
- WO 2005035791 A1
- US 7708949 B
- US 20110250597 A
- JP 2004290977 A
- JP 2004351417 A
- US 20120122714 A
- US 20110000560 A
- US 201001376163 A
- US 20100022414 A
- US 20080003142 A

**Non-patent literature cited in the description**

- *Adv. Mat.,* 2004, vol. 16 (19), 1685-1706
- *J. Biol. Chem.,* 1969, vol. 244, 3019-3028